# EUROPEAN PATENT APPLICATION

(11) **EP 1 106 620 A2**
(43) Date of publication of application: **13.06.2001**
(21) Application number: 00106507.7
(22) Date of filing: 25.03.2000
(51) Int. Cl.: C07G 17/00, A61K 35/14

(54) **Method for obtaining components from cultured leucocytes**

(30) Priority: 09.12.1999 JP 35051899
(71) Applicant: Matsumoto, Tsukasa, Tokyo 158-0084 (JP)
(72) Inventor: Matsumoto, Tsukasa, Tokyo 158-0084 (JP)
(74) Representative: Müller, Enno, Dipl.-Ing.

(57) **Abstract**

A method for obtaining the leucocyte components from human blood, comprises (A) a first step for fracturing the cell membrane of leucocytes of the human blood by using a freezing and defrosting work, a supersonic application, a laser irradiation, an osmotic pressure changing work, a vacuum chamber, or the like and (B) a second step for separating and collecting the leucocyte-components from the blood liquid resulted from the first step, containing the leucocytes with fractured cell membranes, by means of a centrifugal precipitation technique or an electrophoresis technique.

Thus separated and collected leucocyte components is respectively subjected to various therapeutic tests using blood samples collected from patients suffering from various diseases to know the therapeutic effects.

## Description

### BACKGROUND OF THE INVENTION

### 1. Field of the Invention

The present invention relates to a method for obtaining components by fracturing the cell membrane of leucocytes in human blood, and separating and collecting the leucocyte components, individually.

### 2. Prior Art

Conventionally, it has been commonly known that erythrocytes of human blood mainly act as carriers for carrying a large amount of oxygen and carbon dioxide at a high speed, and leucocytes act as phagocytes against various bacteria. However, in practical scenes, erythrocytes also crowd at the inflamed part where a great deal of bacteria exist. It seems that thus crowded erythrocytes act as any biological function in the inflamed part. In addition to this phenomenon, the biological correlation between erythrocytes and leucocytes in such inflamed and bacterial affected parts has not been clearly known. The inventor of the present application previously submitted the patent application titled "Method for fractionating red blood cells" Japanese Patent Application No. Tokugan-Hei 8-215552/1996, corresponding to European Patent Application No. 97102952.5 titled "Method for fractionating red blood cells and antibacterial materials or bacterial proliferation inhibitors produced thereby". The inventor observed the biological function between the erythrocytes and bacteria included in respective three layers fractionated in accordance with the previously applied method.

In detail, the three erythrocyte-samples from the fractionated three layers; i.e., top, middle, and bottom layers, were respectively added into liquid culture medium, and then bacteria sample was inoculated into all of the culture medium to observe the function of live erythrocytes against the inoculated bacteria. In the top layer the bacteria were surrounded with the erythrocytes and thus their bacterial proliferation were inhibited. Although the top layer included leucocytes, these leucocytes did not provide any phagocytosis to the bacteria. The erythrocytes included in the middle layer did not show any aggressive motions towards the bacteria, but the bacterial proliferation were inhibited. The middle layer also included a small amount of leucocytes, which did not show any motions towards the bacteria. The erythrocytes included in the bottom layer did not show any aggressive motions towards the bacteria. Thus, the bacterial proliferation were observed. The bottom layer did not include leucocytes at all.

Since the number of leucocytes in human blood are remarkably increased when bacterial inflammation causes or the size of specific leucocyte is extremely enlarged in bacterial inflamed part, it has been conventionally realized that these phenomena are resulted from the phagocytosis of leucocytes. However, as disclosed in the previously applied invention "Method for fractionating red blood cells" the observation on the action and change of live erythrocytes and leucocytes around bacteria proves that conventional knowledge; i.e., leucocytes only attack bacteria, is not correct.

According to conventional knowledge, erythrocytes comprise single type blood cells having a uniform figure and the same characteristics, while leucocytes comprise several different real cells having nucleus and cytoplasm. Conventionally, leucocytes are classified depending on appearance and staining property into five groups, neutrophil leucocyte, eosinophile leucocyte, basophilic leucocyte, lymphoid cell, and monocyte as disclosed in "Seikagaku Jiten (Encyclopedia of Biochemistry)" published by Tokyo Kagakudojin. Conventional method for laboratory tests of erythrocytes and leucocytes have been performed as following steps. One drop of blood is placed on a glass plate and is spread into a thin layer using the end of another glass plate. After drying, this blood sample is stained with various staining agents and then the stained sample is observed through an optical microscope. This conventional method, however, leads to following three defects.
(1) As the blood sample is dried, the cells are mummified. Therefore this conventional method is too primitive and coarse to observe such delicate, soft, and volatile cells as the erythrocytes and leucocytes.
(2) In the staining technique, the blood cells and components difficult of staining cannot be observed at all. The part that does not stain easily is ignored and the part that stains easily is over-stained darkly. Accordingly, minute changes cannot be observed.
(3) In biological researches, a phenomenon should be observed from the beginning to the end covering as long a period as possible. The biological truth cannot be known unless the state changing with time is grasped.

Conventionally, Ficoll-Conray method has been known to separate and collect lymphoid cells from leucocytes. Thus collected lymphoid cells have been used in various manners to study the immune system about the leucocytes in human blood. For example, the inventor of the present application found out that remarkable effects were not resulted when the lymphoid cells obtained from a healthy person were administered to the rheumatics, but remarkable effects were resulted when cultured leucocytes of a healthy person were administered to the same patient. Further, the inventor has continuously researched on clinical cases of many rheumatics administered with the cultured leucocytes, and confirmed the superior effects in medical treatment for the rheumatics. The inventor reported and published such therapeutic effects at many academic meetings and in bulletins; as an example, International Rheumatism Conference 1981 held in Paris.

It has been assumed that stale cells are broken into water-soluble fine pieces and transported by flowing blood towards liver and kidney. Although the liver and kidney act as the final wastes treatment, the processes of decomposition and water-solubilization of stale cells prior to the final wastes treatment are not clarified. The inventor used the bottom layer (excluding leucocytes) of the fractionated three layers provided by "Method for fractionating red blood cells" to observe the wastes treatment function of leucocytes. In detail, the bottom layer was added with frozen leucocytes or live leucocytes of the same blood type, and then cultured. As a result, this cultured bottom layer showed that the blood cells of the bottom layer were changed to minute and water-soluble particles rather than the non-treated sample free from leucocytes. Additionally, this effect appeared remarkably in the case of using the frozen leucocytes rather than the live leucocytes. This phenomenon means following two functions. First, leucocytes have the function for treating stale or perished erythrocytes as wastes. The second function is anticipated from the phenomenon of the frozen leucocytes having superior effect to live leucocytes. Some components spread out of the leucocytecells fractured during freezing step may act as agents which accelerate the decomposition and water-solubilization of stale or perished erythrocytes.

As discussed above, many clinical tests proved that the cultured leucocytes obtained from a healthy person had effect on specific diseases such as rheumatic disease. However, in actual therapeutic scenes, the cultured leucocytes should be subjected to various tests, for example, the blood check for AIDS virus, prior to administration, and further, should be prepared and stored in a specially controlled space such as an aseptic culture room with meticulous care. Thus therapeutic cost will become high. As an example in an actually performed test-therapy, an administration of cultured leucocytes is performed per four weeks; one course includes six times of administrations; and at least four courses are desired. One administration needs about Yen 50,000 (US$ 550), and thus totally four courses need about Yen 1,200,000 (US$ 13,200). This cost is extremely high for ordinary patients, and therefore on the present stage, actual therapies use steroid drugs which produce harmful aftereffects and anti-inflammatory or analgesic agents which produce temporary pain-free effect.

According to the above observations, the inventor concludes that the correlation between the leucocyte components and the erythrocytes, the therapeutic function of the leucocyte components for various bacteria, and the function of the leucocyte components for treating stale or perished cells should be clarified in order to produce a new drug which is free from harmful aftereffects produced by antibiotics and steroid drugs. Further such leucocytes induced drugs are expected to be applied to the therapy for various cancers, hepatic disease, and renal disease at a low cost. If hepatic disease and renal disease are healed or improved by such new drugs, it is expected to decrease lever and kidney transplant operations which are risky, complicated and high cost operations.

### SUMMARY OF THE INVENTION

It is therefore a primary object of the present invention to provide a method for fracturing the cell membrane of leucocytes in human blood to separate and collect leucocyte components.

Another object of the present invention is to provide a method to separate and collect the leucocyte components under the nearlive condition.

In order to accomplish the above objects, the present invention provides a method comprising (A) a first step for fracturing the cell membrane of leucocytes in human blood by physical means, and (B) a second step for separating and collecting the leucocyte components from the blood liquid resulted from the first step, containing the leucocytes with fractured cell membranes, by means of a centrifugal precipitation technique or an electrophoresis technique.

Thus separated and collected leucocyte components may be respectively subjected to various therapeutic tests using blood samples collected from patients suffering from various diseases to know the therapeutic effects. One typical example of these therapeutic tests is performed in the following steps.
(1) 5 to 10 ml of blood sample is taken from a patient suffering from cancer, hepatic disease, or renal disease and then separated into three layers; top layer, middle layer, and bottom layer, in the same manner as shown in the method for fractionating red blood cells, disclosed in the prior invention. These three layers are cultured respectively, and their changes are observed through a phase-contrast microscope for a predetermined period. These observed data are recorded by a still camera, video camera, or the like, and used as reference for judging the therapeutic effects.
(2) The three layers are also obtained from the same patient's blood in the same fractionating method, and then added with one group of the leucocyte components separated from a healthy person's blood. The three layers are cultured and the changes of erythrocytes are observed through the same phase-contrast microscope at a predetermined time interval. According to the above works (1) and (2), it is possible to judge which leucocyte component has the therapeutic effect on which kind of diseases.

The physical means used in the first step (A) for fracturing the cell membranes of leucocytes may be selected from (a) a supersonic method for applying the supersonic of 1 MHz to 50 MHz to the blood liquid containing leucocytes to fracture the cell membranes of leucocytes by the vibration caused by the supersonic; (b) a laser method for irradiating the laser of 10 to 100 mW, 50/cm² for several seconds to several minutes (about 3 minutes) to the same point in the blood liquid containing leucocytes to fracture the cell membranes; (c) an osmotic pressure method for changing the osmotic pressure of the blood liquid containing leucocytes to fracture the cell membranes; (d) a freezing and defrosting method for freezing the blood liquid containing leucocytes at the temperature range from -5 °C to the absolute zero point and then defrosting this frozen liquid at a room temperature (about 20 °C) to fracture the cell membranes; and (e) a vacuum method for rapid-reducing the pressure in a vacuum chamber to fracture the cell membranes of the blood liquid containing leucocytes set in the chamber.

The second step (B) for separating and collecting the leucocyte component includes a centrifugal precipitation technique which stirs the blood liquid containing the leucocytes with cell membranes fractured by the first step (A), and then separates the stirred liquid into multiple layers corresponding the leucocyte components by the centrifugal precipitation work. Alternatively, the second step (B) includes an electrophoresis technique which separates the blood liquid containing the leucocytes with fractured cell membranes by the first step (A) into multiple parts corresponding the leucocyte components by the electrophoresis work. Finally, the separated layers or parts are collected separately.

The first step (A) may use the cultured leucocytes obtained from the specific persons who are judged healthy through predetermined health checks.

These and other objects and many of the attendant advantages of the present invention will be readily appreciated as the same becomes better understood by reference to the following detailed description when considered in connection with the accompanying drawings.

### BRIEF DESCRIPTION OF THE DRAWING

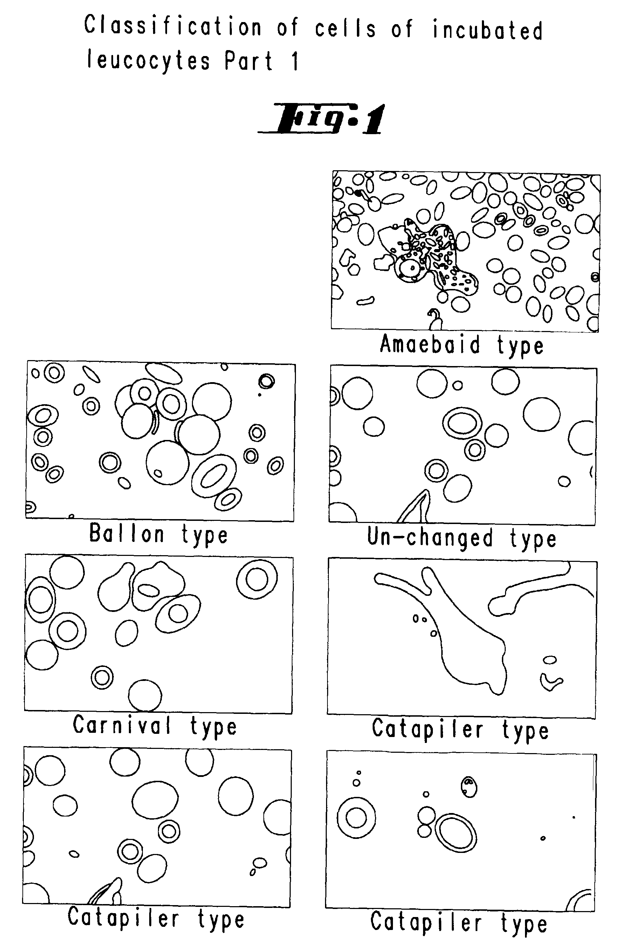

Fig. 1 is a photographic data observed through a phase-contrast microscope showing the leucocytes cultured for 48 hours;

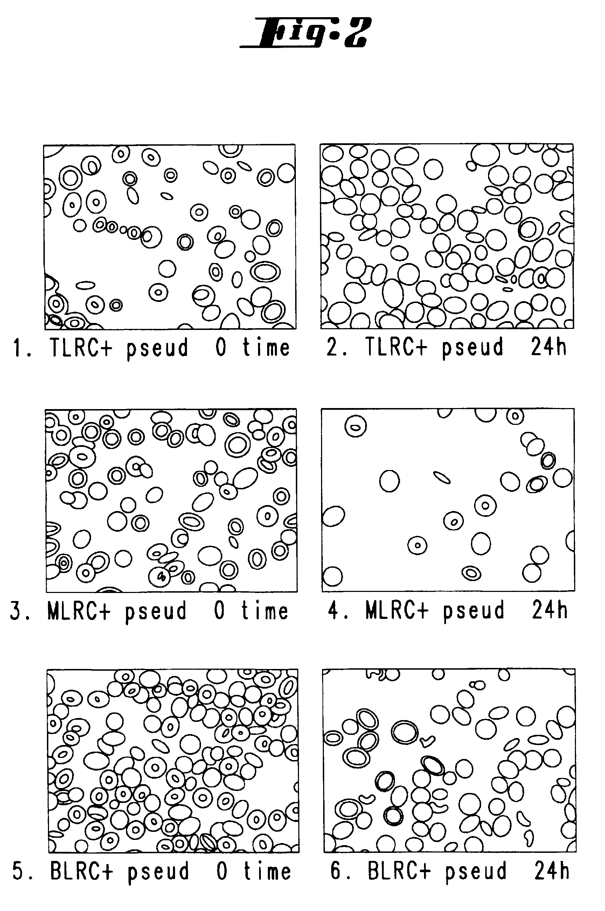

Fig. 2 is another photographic data observed through a phase-contrast microscope showing fractionated blood samples by the method for fractionating red blood cells of human blood which include one series showing immediately after inoculated with bacteria, and the other series showing 24 hours later; and

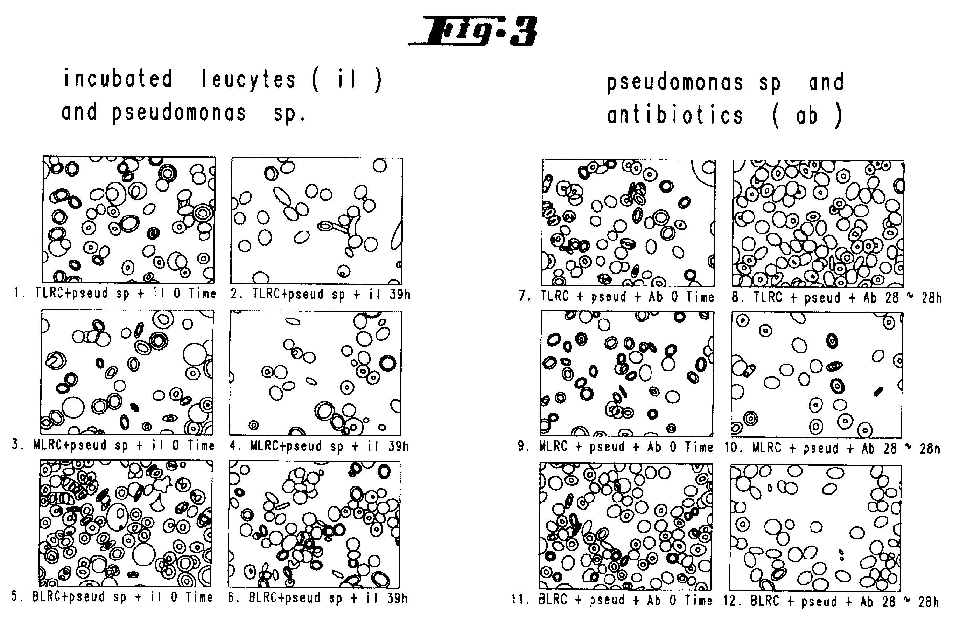

Fig. 3 is other photographic data observed through a phase-contrast microscope showing fractionated blood samples by the method for fractionating red blood cells of human blood which include one series showing immediately after inoculated with bacteria and incubated leucocytes or antibiotics, and the other series showing 39 hours later or 28 to 29 hours later.

### DETAILED DESCRIPTION OF THE PREFERRED EMBODIMENT

According to one preferred embodiment of the present invention, leucocytes obtained from a healthy person are cultured, and then leucocyte components are separated and collected. Thus collected leucocyte components are added to various blood samples obtained from various patients suffering from diseases in order to find and judge therapeutic effects. Detailed process of the preferred embodiment will be disclosed as follows.

First, leucocytes are separated from human blood containing all blood ingredients in a typical conventional manner. 200 ml of human blood obtained from a healthy person (who was passed through all possible health checks and blood checks at present stage) is added into 300 ml of Dextran-70 (commercially available by Tokyo Kasei Chemical, Tokyo Japan) aqueous solution and mixed thoroughly. This mixture is remained stationarily for one hour at a room temperature of 25°C or higher, or for 80 minutes at a temperature range between 20 and 25 °C. During one hour, or 80 minutes, the leucocytes and part of the erythrocytes are concentrating in the surface region of the mixture, and this concentrated substance is collected and subjected to a centrifugal precipitation step at 1000 rpm. Thus produced clear supernatant is thrown away. RLB (hypotonic solution, produced by Harajuku Clinic, Tokyo Japan) is firstly added to the remaining solution, and after 30 seconds, BELMAR (hypertonic solution, produced by Harajuku Clinic, Tokyo Japan) is added to it. By this process, white, cheese-like layer is formed. If this white layer is not successfully formed by this first attempt, then the addition of the hypotonic solution and the hypertonic solution may be repeated until the white layer is formed. This white layer is subjected again to the centrifugal precipitation at 1000 rpm and its clear supernatant is removed. The precipitate is added with RPMI-1640 (tissue culture medium, produced by GIBCO BRL, USA) and 5% FBS (fetal bovine serum), and they are remained in an incubator under the condition of 5% carbon dioxide, at 37°C for 48 hours.

After 48 hours, the incubated sample is observed through a phase-contrast microscope (Nikon TMS-F MFA2010). The leucocytes after 48 hours incubation change into the following five shapes as shown in Fig. 1.

Fig. 1 shows five type photographs representing (1) carnival type cells (++++ ~ v), (2) cells with no change (++ ~ +++), (3) balloon type cells (++ ~ +++), (4) caterpillar type cells (++ ~ +++), and (5) amoeba type cells (+), in order of counted numbers. These name of five types are given by the present inventor according to their appearances.

The leucocyte solution after 48 hours incubation is further subjected to a centrifugal precipitation at 1000 rpm, and its clear supernatant is removed. This remained precipitate is washed by 20 to 50 ml of Physiosol No. 3 and centrifuged. These washing and centrifugal precipitation steps are repeated twice. Thus obtained leucocyte precipitate is additionally added with 100 to 200 ml of Physiosol No. 3, and maintained at 5°C or lower. This is designated Sample A. In the actually performed therapy for rheumatoid arthritis, the inventor infuses this Sample A intravenously within 24 hours.

Second, in order to clarify the therapeutic effects owing to the cultured leucocytes (Sample A), one typical test is performed as follows. Three fractionated blood samples provided by the method for fractionating erythrocytes are respectively inoculated with bacteria and their changes are observed. In detail, 10 ml of blood sample (including all blood ingredients) is taken from a healthy person. This blood sample is mixed with 15 ml of 7% Dextran aqueous solution and stirred sufficiently. Then this mixture is remained stationarily for 60 to 75 minutes at a room temperature from 20 to 25°C. This mixture is separated into three layers; i.e., top layer, middle layer, and bottom layer. Respective blood samples are separately taken from these three layers by means of a pipette. Four to six drops of each blood sample are added into a flask (produced by Costar) including 3 ml of a liquid culture medium RPMI-1640. Thus prepared blood samples are designated Sample B (containing types T, M, and B). Additionally, these cultured samples in the flasks are inoculated with bacteria such as pseudomonas sp. and remained in an incubator under the condition of 5% carbon dioxide, at 37°C.

The changes of the Samples B, (types T, M and B) are observed through the phase-contrast microscope at immediately after the bacteria incubation, and after 24 hours. Thus resulted photographic data are shown in Fig. 2. The photograph No. 1 denotes the top layer of the fractionated red blood cells immediately after the bacteria incubation. The photograph No. 2 denotes the top layer after 24 hours. The photograph No. 3 denotes the middle layer immediately after the bacteria incubation. The photograph No. 4 denotes the middle layer after 24 hours. The photograph No. 5 denotes the bottom layer immediately after the bacteria incubation. The photograph No. 6 denotes the bottom layer after 24 hours. These photographic data indicate that the bacterial proliferation in the top and middle layers are inhibited to a certain extent owing to the leucocytes included in these layers inherently, while the bacterial proliferation in the bottom layer is not inhibited.

Next, the three fractionated blood samples (corresponding to Sample B) are inoculated with bacteria such as pseudomonas sp. and simultaneously mixed with the cultured leucocytes (corresponding to Sample A). The changes of the Samples B (types T, M and B) are observed through the phase-contrast microscope at immediately after the bacteria incubation and the leucocytes administration, and after 39 hours. Thus resulted photographic data are shown in Fig. 3. The photograph No. 1 denotes the top layer of the fractionated red blood cells immediately after the bacteria incubation and the leucocytes administration. The photograph No. 2 denotes the top layer after 39 hours. The photograph No. 3 denotes the middle layer immediately after. The photograph No. 4 denotes the middle layer after 39 hours. The photograph No. 5 denotes the bottom layer immediately after. The photograph No. 6 denotes the bottom layer after 39 hours. The photographs No. 7 to No. 12 correspond to No. 1 to No. 6 except for replacing the leucocytes administration with the antibiotics administration, respectively.

These photographic data No. 1 to No. 6 indicate that the bacterial proliferation in the bottom layer subjected to the cultured leucocytes administration is slightly inhibited in addition to the top and middle layers. As shown in No. 7 to No. 10, although the bacterial proliferation in the top and middle layers is remarkably inhibited owing to the antibiotics, such inhibition effect is gradually degraded as time passes. No. 11 and No. 12, the bottom layer samples, do not show such inhibition effect at all even though the antibiotics are added to the bottom layer samples. This phenomenon can not be explained by the conventional MIC (minimum inhibitory concentration) theory. Since this conventional theory is based on various experiments in vitro using glass vessels, the actual effects caused by antibiotics in vivo; i.e., human body, can not be correctly explained by this conventional theory.

The administration of cultured leucocytes allows the erythrocytes to enhance their inhibition effect for bacterial proliferation at least as same as antibiotics. Particularly, as time passes, the inhibition effect owing to the antibiotics is degraded rather than the cultured leucocytes. As is well known, the harmful aftereffects are caused by using antibiotics and some bacteria will acquire the resistance to the specific antibiotics which have been frequently used. The leucocytes are inherently existed in human body and known as a key-element for controlling spontaneous cure. Although the above described therapeutic test shows one example which uses one of bacteria to clarify the therapeutic effect caused by the cultured leucocytes, the same process of this test can also judge the therapeutic effects to various diseases such as various virus diseases, cancers, hepatic disease, renal disease and so on by using blood samples obtained from the patients suffering from these diseases. For example, in the case of patients suffering from type C hepatitis and dialysis, when the cultured leucocytes obtained from the patients themselves are added to their blood samples, their erythrocytes become poor in their activity and quality within 4 or 5 days. When the cultured leucocytes of a healthy person are added to these patients' blood samples, their erythrocytes maintain their initial activity and quality for a relatively long period. Additionally, the administration of the healthy person's cultured leucocytes to these patients' blood samples enhances the inhibition effect for bacterial proliferation remarkably. The similar effects are shown in the case of patients suffering from cancer and rheumatism.

As shown in Fig. 1, the 48 hours cultured leucocytes are classified into five shape groups in accordance with the microscopic observation. The cultured leucocytes used for the currrently performed therapy include all components of the five groups. If the component of the leucocytes having the therapeutic effects is specified, then this specified component will be chemically synthesized or extracted so as to realize superior drugs free from harmful aftereffects caused by currently used antibiotics. In other words, the object of the present invention is to provide a method for separating a single component from the leucocytes prior to the research on the relation between the leucocyte component and the therapeutic effect.

In the method for separating and collecting the leucocyte components from the above described cultured leucocytes according to one preferred embodiment of the present invention, the leucocyte cells are fractured as follows. The cultured leucocyte sample is frozen at the temperature range from -5°C to -72°C, preferably -20°C, and remained under such freezing condition for 10 days. This frozen leucocyte sample is defrosted at a room temperature within 1 to 3 hours. The defrosted leucocyte sample is subjected to a centrifugal precipitation from a low speed of 5 to 50 rpm to a high speed of 800 rpm. This centrifugal precipitation forms separated layers. Alternatively, the defrosted leucocyte sample is subjected to an electrophoresis process. This process also forms separated parts. Thus separated layers or parts are individually collected.

The physical means for fracturing the cell membranes of leucocytes is not limited to the above described freezing and defrosting process, but can be also selected from a supersonic method for applying the supersonic of 1 MHz to 50 MHz to the blood liquid containing leucocytes to fracture the cell membranes of leucocytes by the vibration caused by the supersonic; a laser method for irradiating the laser of 10 to 100 mW, 50/cm² for several seconds to several minutes (about 3 minutes) to the same point in the blood liquid containing leucocytes to fracture the cell membranes; an osmotic pressure method for changing the osmotic pressure of the blood liquid containing leucocytes to fracture the cell membranes; and a vacuum method for rapid-reducing the pressure in a vacuum chamber to fracture the cell membranes of the blood liquid containing leucocytes set in the chamber.

The collected component belonging to each of the separated layers or parts is subjected to the above described therapeutic tests to clarify its therapeutic effects for various bacteria and diseases and the functions for treating stale or perished cells. The therapeutic effects means that the spontaneous curing ability caused by the correlation between leucocytes and erythrocytes to inhibit the proliferation of bacteria or virus is returned to the normal person's level. The diseases disclosed above means various cancers, hepatitis, nephritis, rheumatoid arthritis, various inflammatory diseases, infectious diseases of virus or rickettsias, AIDS, or the like. In order to find which leucocyte component enhances the activity of erythrocytes remarkably, each one of the components separated and collected by the method according to the present invention is added to the blood samples obtained from the patients suffering from these diseases. Since it is possible to generate multiplier effects, two or three components may be combined to improve therapeutic effects.

As is clear from the above description, the method provided by the present invention can fracture the leucocyte-cell membrane to separate and collect the leucocyte components. If the component of the leucocytes having the therapeutic effects is specified, then this specified component will be chemically synthesized or extracted so as to realize superior drugs free from harmful aftereffects caused by currently used antibiotics. Particularly, in C type hepatitis, autoimmune hepatitis, and renal diseases, the correlation between leucocytes and erythrocytes is not normally performed and the activity of erythrocytes is degraded. This causes that the leucocytes' function for treating stale or perished cells becomes poor and these stale or perished cells are accumulated in hepatic and renal tissues. This accumulation results in the diseases of kidney and liver. If the function of the leucocyte components for treating stale or perished cells is clarified, a new drug which is free from harmful aftereffects produced by antibiotics and steroid drugs will be produced.

As many apparently widely different embodiments of this invention may be made without departing from the spirit and scope thereof, it is to be understood that the invention is not limited to the specific embodiments thereof except as defined in the appended claims.

## Claims

1. A method for obtaining the leucocyte components from human blood comprising;
(A) a first step for fracturing the cell membrane of leucocytes of the human blood by physical means, and
(B) a second step for separating the leucocyte components from the blood liquid resulted from the first step, containing the leucocytes with fractured cell membranes, to collect the separated layers or parts individually.

2. The method for obtaining the leucocyte components from human blood according to claim 1, wherein the physical means used in the first step (A) for fracturing the cell membranes of leucocytes is selected from (a) a supersonic method for applying the supersonic of 1 MHz to 50 MHz to the blood liquid containing leucocytes to fracture the cell membranes of leucocytes by the vibration caused by the supersonic; (b) a laser method for irradiating the laser of 10 to 100 mW, 50/cm² for several seconds to several minutes (about 3 minutes) to the same point in the blood liquid containing leucocytes to fracture the cell membranes; (c) an osmotic pressure method for changing the osmotic pressure of the blood liquid containing leucocytes to fracture the cell membranes; (d) a freezing and defrosting method for freezing the blood liquid containing leucocytes at the temperature range from -5 °C to the absolute zero point and then defrosting this frozen liquid at a room temperature (about 20 °C) to fracture the cell membranes; and (e) a vacuum method for rapid-reducing the pressure in a vacuum chamber to fracture the cell membranes of the blood liquid containing leucocytes set in the chamber.

3. The method for obtaining the leucocyte components from human blood according to claim 1, wherein the second step (B) for separating the leucocyte component includes a centrifugal precipitation which stirs the blood liquid containing the leucocytes with cell membranes fractured by the first step (A), and then separates the stirred liquid into multiple layers corresponding the leucocyte components by the centrifugal precipitation.

4. The method for obtaining the leucocyte components from human blood according to claim 1, wherein the second step (B) includes an electrophoresis work which separates the blood liquid containing the leucocytes with fractured cell membranes by the first step (A) into multiple parts corresponding the leucocyte components by the electrophoresis work.

5. The method for obtaining the leucocyte components from human blood according to claim 1, wherein the first step (A) uses the cultured leucocytes obtained from the specific persons who are judged healthy through predetermined health and blood checks.

6. The method for obtaining the leucocyte components from human blood according to claim 1, further comprising a step for finding therapeutic effects owing to the separated and collected leucocyte components, including various therapeutic tests using blood samples collected from patients suffering from various diseases.
